# EUROPEAN PATENT SPECIFICATION

(11) **EP 0 799 818 B1**
(45) Date of publication and mention of the grant of the patent: **02.05.2002**
(21) Application number: 97201945.9
(22) Date of filing: 11.10.1994
(51) Int. Cl.: C07C 211/54, G03G 5/06

(54) **Benzidine derivatives and electrophotosensitive material using the same**
Benzidinderivate und elektrophotoempfindliches Material unter Verwendung derselben
Dérivés benzidines et matériau électrophotosensible les utilisant

(30) Priority: 13.10.1993 JP 25609093; 14.10.1993 JP 25720793; 14.10.1993 JP 25720993; 03.12.1993 JP 30443893; 08.04.1994 JP 7042294; 08.04.1994 JP 7042394; 08.04.1994 JP 7042494; 08.04.1994 JP 7042794
(43) Date of publication of application: 08.10.1997
(62) Divisional of application: 94307440.1
(73) Proprietor: KYOCERA MITA CORPORATION, Chuo-ku , Osaka 540-8585 (JP)
(72) Inventor: Mizuta, Yasufumi, Tamatsukuri, Chuo-ku, Osaka 540 (JP); Tanaka, Masashi, Tamatsukuri, Chuo-ku, Osaka 540 (JP); Muto, Nariaki, Tamatsukuri, Chuo-ku, Osaka 540 (JP); Fukami, Toshiyuki, Tamatsukuri, Chuo-ku, Osaka 540 (JP); Nakamori, Hideo, Tamatsukuri, Chuo-ku, Osaka 540 (JP); Kakui, Mikio, Tamatsukuri, Chuo-ku, Osaka 540 (JP); Saito, Sakae, Tamatsukuri, Chuo-ku, Osaka 540 (JP); Shiomi, Hiroshi, Tamatsukuri, Chuo-ku, Osaka 540 (JP); Sumida, Keisuke, Tamatsukuri, Chuo-ku, Osaka 540 (JP); Uchida, Maki, Tamatsukuri, Chuo-ku, Osaka 540 (JP)
(74) Representative: W.P. THOMPSON & CO.

(56) References cited:
- EP-A- 0 318 916
- US-A- 4 933 245
- DATABASE WPI Week 9051 Derwent Publications Ltd., London, GB; AN 90-380967 XP002128182 & JP 02 277071 A (CANON KK), 13 November 1990 (1990-11-13)

## Description

### BACKGROUND OF THE INVENTION

The present invention relates to novel benzidine derivatives which are suitably used as charge transferring materials, particularly hole transferring materials in applications such as solar batteries, electroluminescent devices, electrophotosensitive materials and the like, and electrophotosensitive materials using the same.

As charge transferring materials, there have been known various organic compounds such as carbazole compounds, oxadiazole compounds, pyrazoline compounds, hydrazone compounds, stilbene compounds, phenylenediamine compounds, benzidine compounds and the like.

These charge transferring materials are normally used in a state where they are dispersed in a membrane of a suitable binding resin. In case of the electrophotosensitive material, for example, so-called organic photosensitive materials (OPC) such as single-layer type electrophotosensitive materials comprising a single-layer type photosensitive layer wherein the above charge transferring material and a charge generating material which generates a charge due to light irradiation are dispersed in a binding resin, multi-layer type electrophotosensitive materials comprising a charge transferring layer containing the above electric transferring material and a charge generating layer containing a charge generating material, etc. are normally used. Such an organic photosensitive materials have an advantage that they are superior in processability and can be easily produced, and which offers a great degree of freedom for design of performance.

Among these compounds, there can be suitably used a 3,3'-dimethylbenzidine derivative represented by the formula: 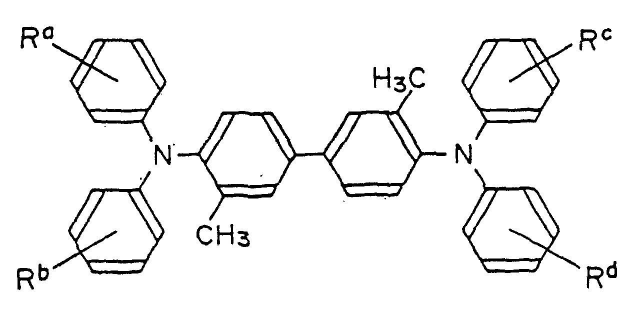 wherein R^{a}, R^{b}, R^{c} and R^{d} are same or different and indicate a hydrogen atom, a lower alkyl group, a lower alkoxy group or a chlorine atom, which belongs to the benzidine derivative, because of its high hole transferring capability and good compatibility with the binding resin (see JP-A-5-210099).

However, the above 3,3' dimethylbenzidine derivatives have such a disadvantage that they have generally low melting points (about 180 °C or less) and therefore the glass transition temperature (Tg) of a membrane comprising the 3,3'-dimethylbenzidine derivative dispersed in the binding resin becomes low, which results in deterioration of durability, heat resistance and the like.

In case of an electrophotosensitive material, for example, the steps of ① charging of the surface of the electrophotosensitive material due to corona discharge, ② formation of a electrostatic latent image due to exposure, ③ toner development of the electrostatic latent image due to adhesion of toner, ④ image transfer of the toner image to a paper and ⑤ removal of the residual toner on the surface of the electrophotosensitive material after image transfer are repeated. For cleaning the residual toner, there can be used a cleaning blade which is pressure-contacted on the surface of the electrophotosensitive material. Therefore, in an electrophotosensitive material using a conventional benzidine derivative, a pressure welding dent is formed at the part where the cleaning blade has been pressure-contacted when the image forming apparatus is stopped, thereby causing various failures of image. Further, when operating the image forming apparatus, the temperature of the interior of the apparatus increases to about 50 °C and therefore a recess is formed on the surface of the electrophotosensitive material, thereby causing various failures of image.

### SUMMARY OF THE INVENTION

The present inventors have striven to increase the melting point of the benzidine derivative, and they carried out molecular designing according to the policy. As a result, it has been found that the melting point of the benzidine derivative can be increased while maintaining high hole transferring capability and good compatability with the binding resin by satisfying at least one of the following conditions, and the present invention has been accomplished.
(1) C₁ to C₆ alkyl groups are introduced in 3,3'- and 5,5'-positions of biphenyl as a center skeleton of the benzidine derivative.
(2) Phenyl groups are bonded to para-positions of two or four outer phenyl groups of the benzidine derivative to form biphenyl groups, thereby imparting stretch of space to a π-electron of conjugate system.

Benzidine derivatives of the present invention can be superior in compatibility with the binding resin while maintaining high hole transferring capability. The derivatives can also improve the durability, heat resistance, etc. of a membrane formed by dispersing the derivative in a binding resin.

The present invention can also provide high-performance electrophotosensitive materials using the above benzidine derivatives as a hole transferring material, the materials can have high sensitivity as well as excellent durability and heat resistance.

Benzidine derivatives of the present invention are represented by the following general formulas (3), (3') and (5) :
I. the general formula (3): 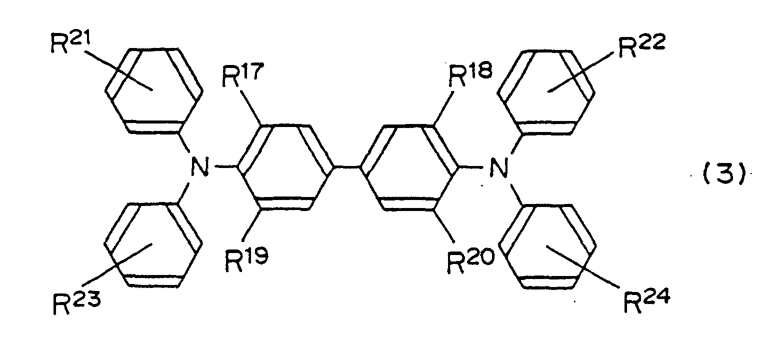 wherein R¹⁷, R¹⁸, R¹⁹ and R²⁰ are the same or different and indicate a C₁ to C₆ alkyl group; R²¹ and R²² are the same or different and indicate a hydrogen atom, or a C₁ to C₆ alkyl group; and R²³ and R²⁴ are the same or different and indicate a phenyl, tolyl, napthyl, bipenyl, anthryl or phenanthryl group or any thereof having a C₁ to C₆ alkyl substituent, or a C₁ to C₆ alkoxy substituent or a halogen substituent, preferably the general formula (3'): 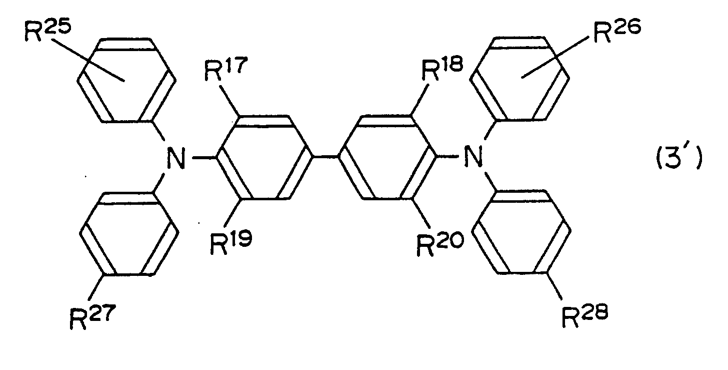 wherein R¹⁷, R¹⁸, R¹⁹ and R²⁰ are as defined above; R²⁵ and R²⁶ are the same or different and indicate a hydrogen atom or a C₁ to C₆ alkyl group; and R²⁷ and R²⁸ are the same or different and indicate;
II. the general formula (5): 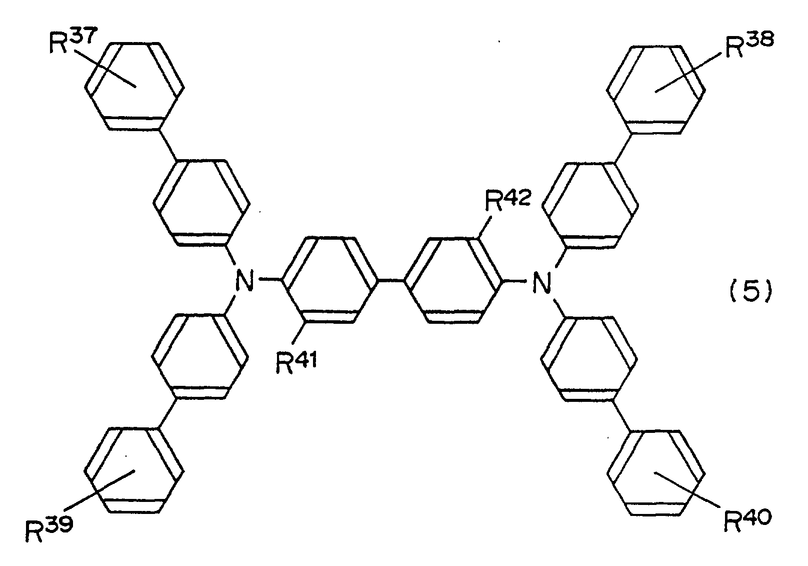
wherein R³⁷, R³⁸, R³⁹ and R⁴⁰ are the same or different and indicate a hydrogen atom or a C₁ to C₆ alkyl group; and R⁴¹ and R⁴² are the same or different and indicate a C₁ to C₆ alkyl group.

The electrophotosensitive material of one embodiment of the present invention comprises a conductive substrate, and a photosensitive layer provided on the conductive substrate, said photosensitive layer containing at least one of benzidine derivatives represented by the above general formulas (3), (3') or (5), preferably as a hole transferring material.

### BRIEF EXPLANATION OF DRAWINGS

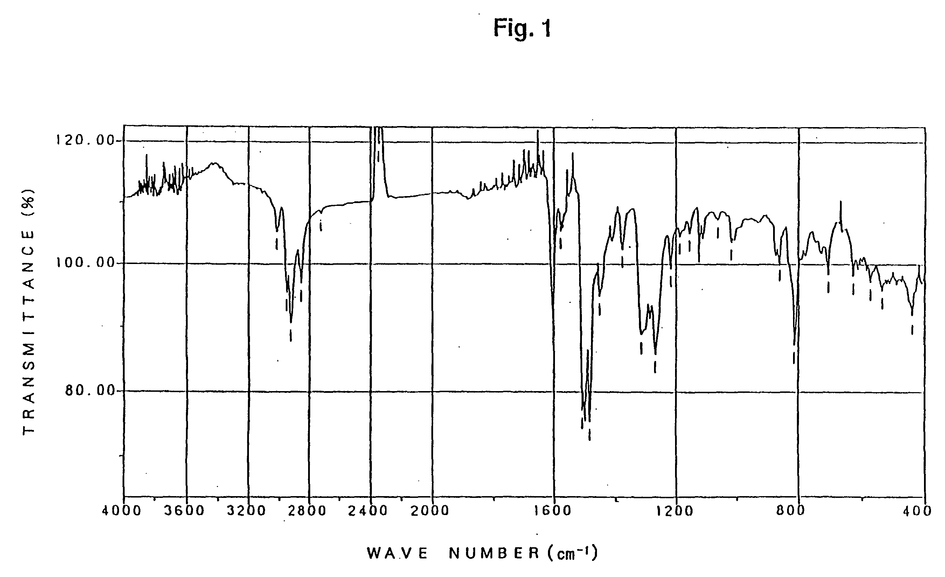

Figs. 1 to 5 are graphs illustrating the results of infrared spectroscopic analysis of benzidine derivatives obtained in Synthesis Examples 1, 2, 3, 6 and 9, respectively.

### DETAILED DESCRIPTION OF THE INVENTION

Examples of the alkyl groups having 1 to 6 carbon atoms are a methyl group, an ethyl group, a propyl group, an isopropyl group, a butyl group, an isobutyl group, a tert-butyl group, a pentyl group, and a hexyl group. Particularly, methyl and ethyl groups are suitably used.

Examples of the alkoxy group having 1 to 6 carbon atoms are a methoxy group, an ethoxy group, a propoxy group, an isopropoxy group, a butoxy group, a tert-butoxy group, a pentyloxy group and a hexyloxy group.

Examples of the halogen atom include chlorine, bromine, iodine and fluorine.

Symmetrical compounds wherein the substituents R²¹ to R²⁴ are the same and their substitution positions are the same and, further, the substituents R¹⁷ to R²⁰ are the same are also included in the benzidine derivative represented by the general formula (3), but unsymmetrical compounds are more preferable, taking performances as the charge transferring material (e.g. compatibility with the binding resin, charge transferring capability, etc.) into consideration. The term "unsymmetrical compound" used herein means those which satisfy at least one of conditions:
① the substituents R²¹ to R²⁴ are different;
② substitution positions of the substituents R²¹ to R²⁴ are different; and
③ the substituents R¹⁷ to R²⁰ are different. Particularly, those which satisfy the condition ① (the substituents R²¹ to R²⁴ are different) are preferable in view of the above performances as the charge transferring material or ease of manufacturing.

Examples of the unsymmetrical benzidine compound include compounds wherein all of the substituents R¹⁷ to R²⁰ are methyl groups and all of the substituents R²¹ to R²⁴ are substituted at the 4-positions of the respective phenyl groups, and the substituents R²¹ and R²² are hydrogen atoms or the same alkyl groups and, further, the substituents R²³ and R²⁴ are the same alkyl groups and are different from the above substituents R²¹ and R²².

Examples of the benzidine derivative represented by the general formula (3) include compounds represented by the following formulas (3-d) to (3-f). As is apparent from these formulas, there are compounds wherein the substituents R²¹ and R²² are hydrogen atoms or compounds wherein the substituents R²¹ and R²² are methyl groups.

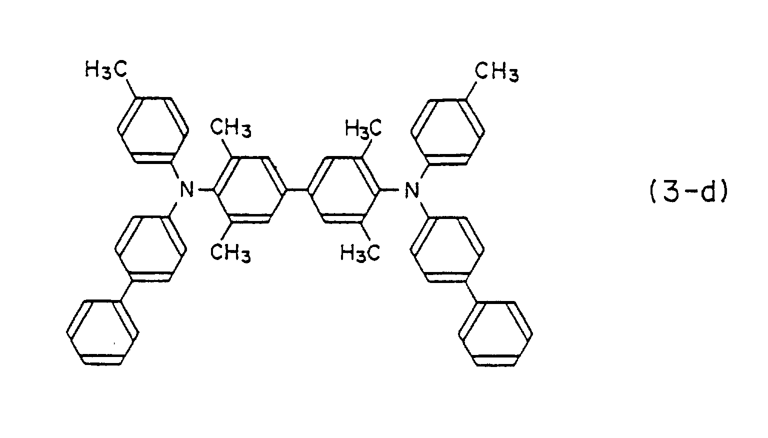

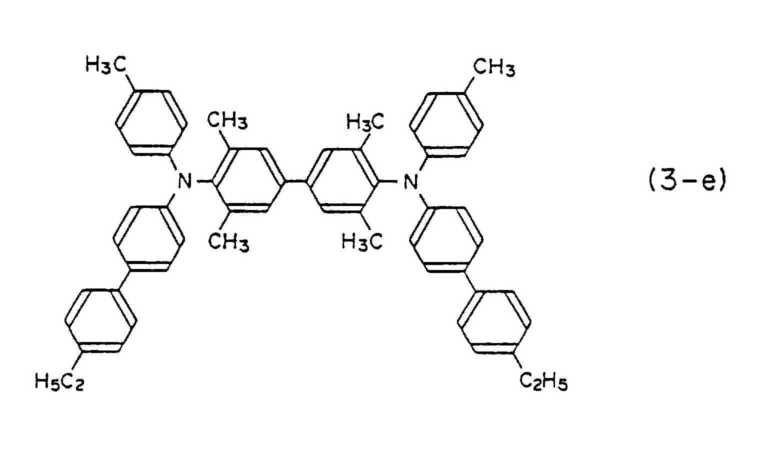

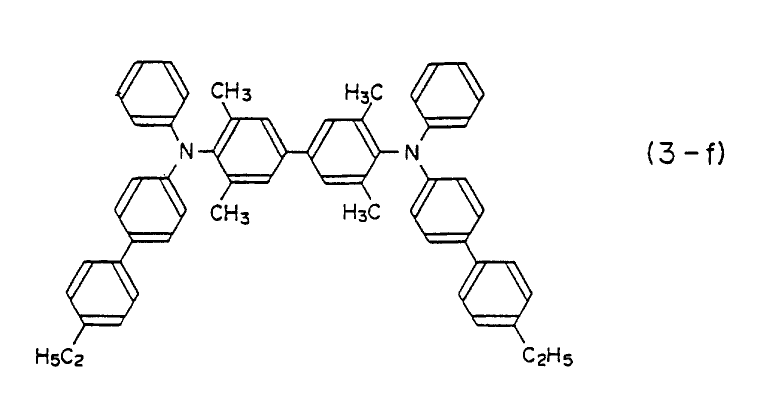

Examples of the benzidine derivative represented by the general formula (5) include compounds represented by the following formulas (5-a) to (5-c).

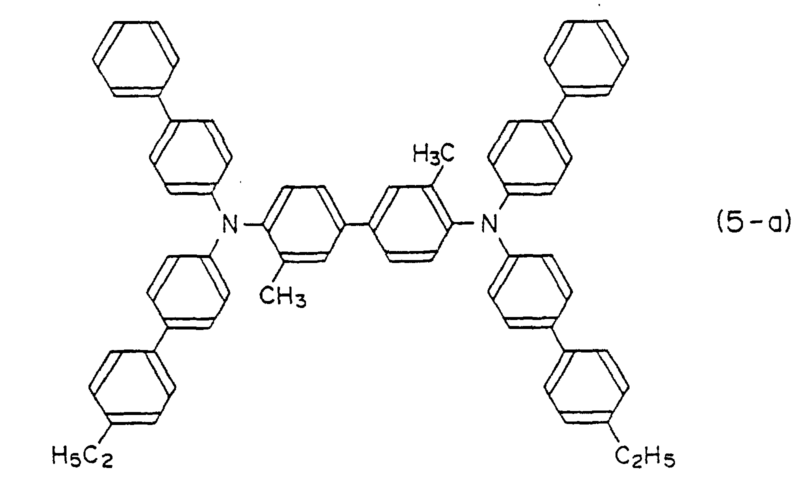

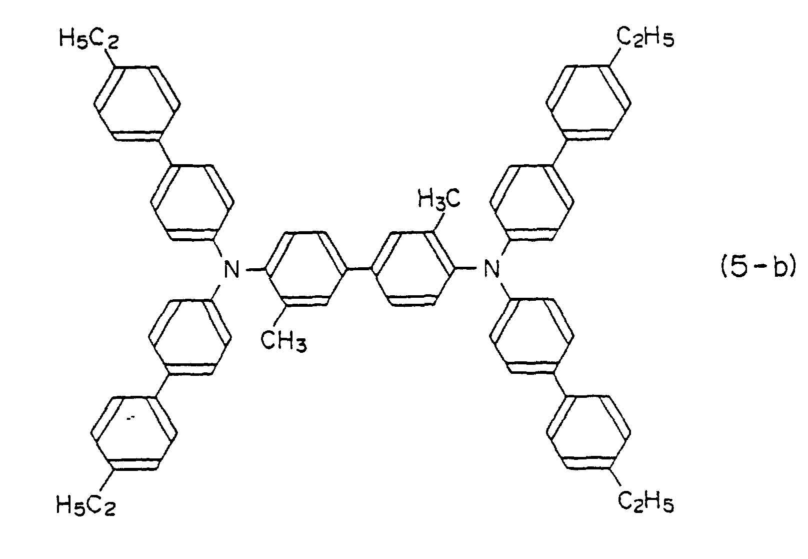

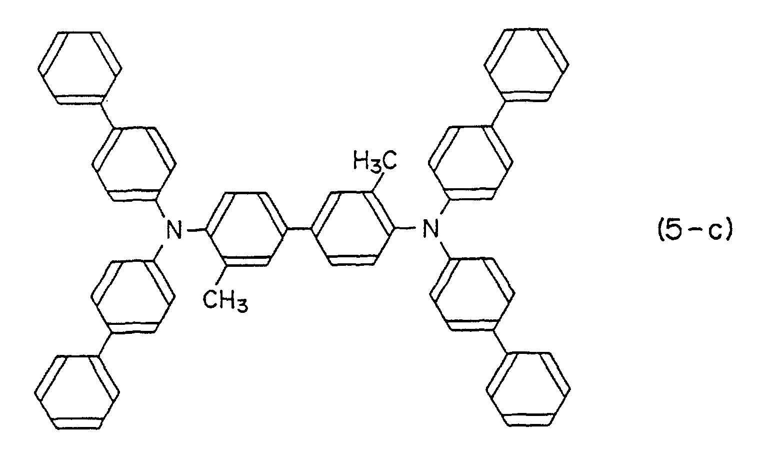

The benzidine derivative of the present invention can be synthesized by various methods. For example, the benzidine derivative of the formula (1-a) below (lying outside the scope of the invention but the subject of Reference Synthesis Example I) can be synthesized according to the following reaction scheme.

Firstly, N,N'-diacetyl-3,3'-dimethylbenzidine represented by the following formula (7) and 3,4-dimethyliodobenzene represented by the formula (8) are mixed together with copper powder, copper oxide or copper halide in a molar ratio of 1:2 and the mixture was reacted in the presence of a basic substance to give N,N'-diacetyl-N,N'-bis(3,4-dimethylphenyl)-3,3'-dimethylbenzidine represented by the formula (9).

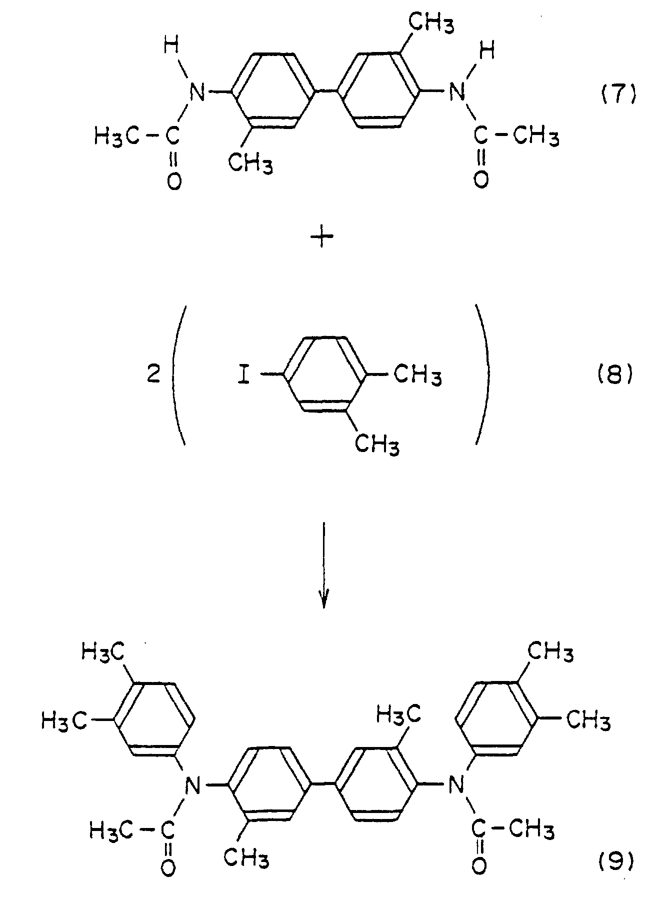

Then, N,N'-diacetyl-N,N'-bis(3,4-dimethylphenyl)-3,3'-dimethylbenzidine represented by the formula (9) is subjected to a deacetylation reaction in the presence of an amide-decomposition catalyst such as acid to give N,N'-bis(3,4-dimethylphenyl)-3,3'-dimethylbenzidine represented by the following formula (10), which is reacted with 4-n-butyliodobenzene in a molar ratio of 1:2 to give a benzidine derivative of the formula (1-a).

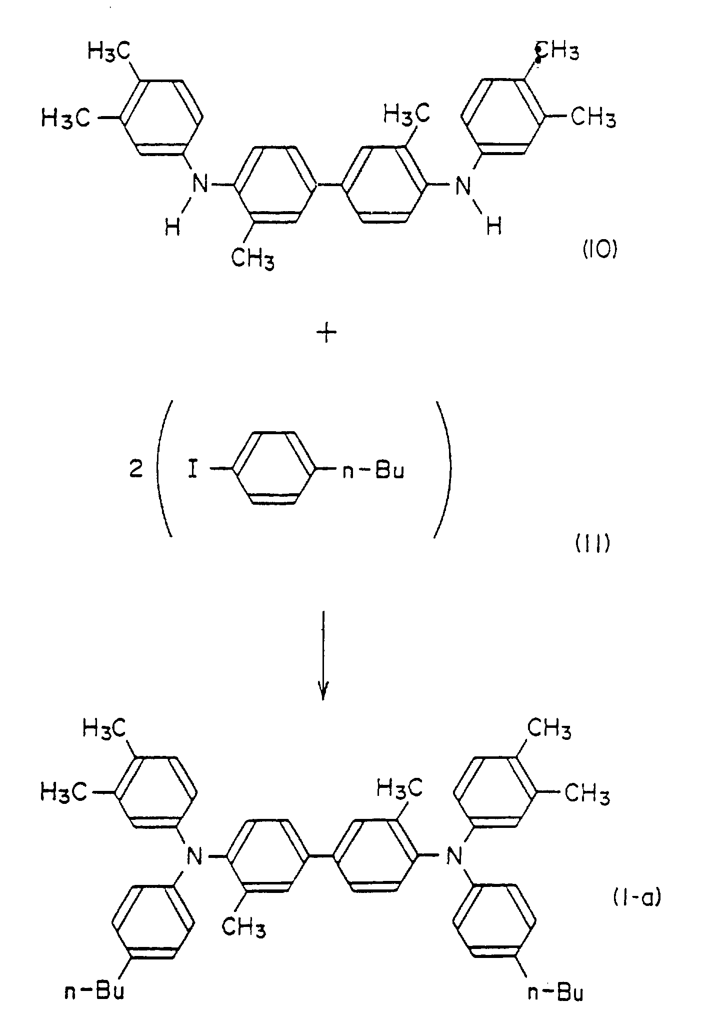

Further, the benzidine derivative of the present invention can also be synthesized by the following reaction scheme in place of the above synthesis method. The following reaction scheme illustrates a synthesis method of the benzidine derivative of the formula (1-d).

Firstly, 4,4'-diiodobiphenyl represented by the following formula (7') and p-isopropylacetanilide represented by the formula (8') are mixed together with copper powder, copper oxide or copper halide in a molar ratio of 1:2 and the mixture was reacted in the presence of a basic substance to give N,N'-diacetyl-N,N'-bis(4-isopropylphenyl)benzidine represented by the formula (9').

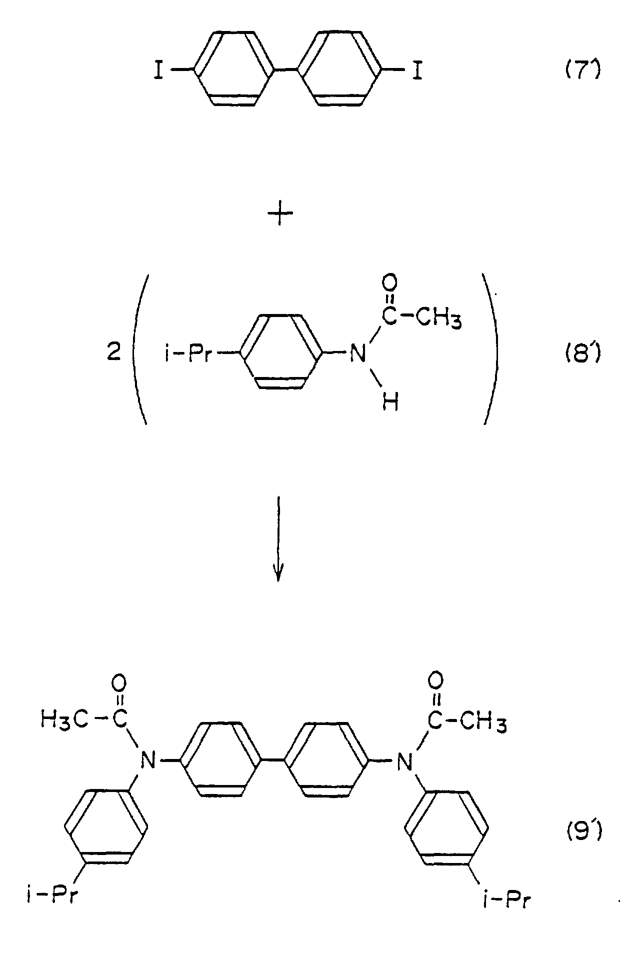

Then, N,N'-diacetyl-N,N'-bis(4-isopropylphenyl)benzidine represented by the above formula (9') is subjected to a deacetylation reaction to give N,N'-bis(4-isopropylphenyl)benzidine represented by the following formula (10'), which is reacted with 2,4-dimethyliodobenzene represented by the formula (10) in a molar ratio of 1:2 according to the same manner as that described above to give a benzidine derivative of the formula (1-d).

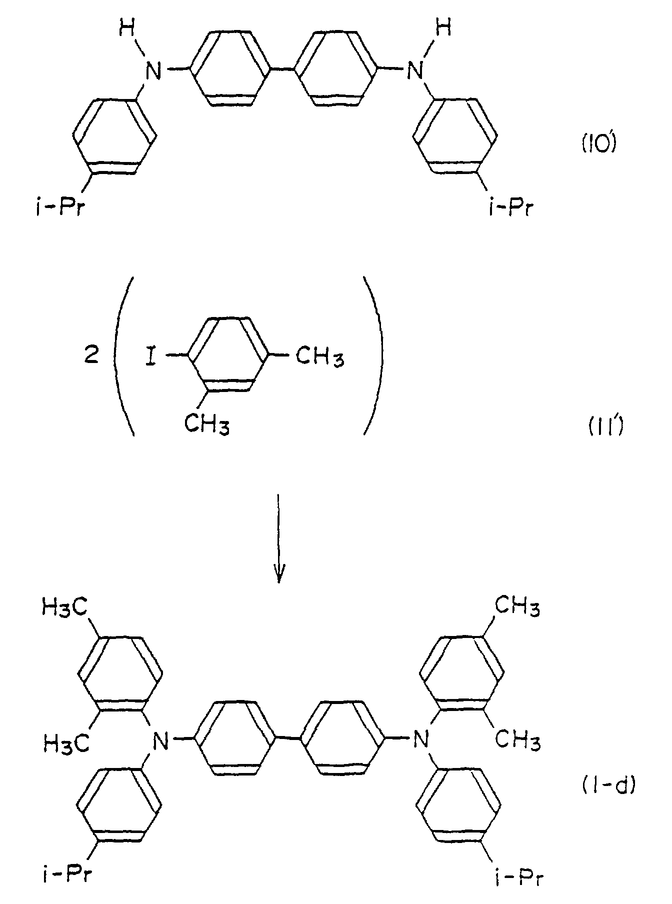

As described above, the benzidine derivative of the present invention is suitably used as a charge transferring material, particularly hole transferring material in applications such as solar battery, electroluminescent material, electrophotosensitive material and the like, and can also be used in other various fields.

The electrophotosensitive material of the present invention comprises a photosensitive layer on a conductive substrate, said photosensitive layer containing one or more sorts of benzidine derivatives represented by the above formulas (3), (3') and (5). The photosensitive layer is classified into two types, i.e. a single-layer type photosensitive layer and a multi-layer type photosensitive layer. The constitution of the present invention can be applied to both photosensitive layers. The benzidine derivative of the general formula (6) is only used for the single-layer type electrophotosensitive material.

The single-layer type photosensitive layer may be obtained by applying a coating solution, which is prepared by dissolving or dispersing at least one of benzidine derivatives represented by the general formulas (3), (3') and (5) and as a hole transferring material, a charge generating material and a binding resin in a suitable solvent, on a conductive substrate using a means such as coating, followed by drying.

Further, the multi-layer type photosensitive layer may be obtained by forming a charge generating layer containing a charge generating material on a conductive substrate using a means such as deposition or coating, applying a coating solution containing at least one of the benzidine derivatives represented by the general formulas (3), (3') and (5) and a binding resin on the charge generating layer using a means such as coating, followed by drying to form a charge transferring layer. To the contrary, a charge transferring layer may be formed on a conductive substrate and then a charge generating layer may be formed thereon.

Non-limited examples of the charge generating material include powder of inorganic photoconductive materials (e.g. selenium, selenium-tellurium, selenium-arsenic, cadmium sulfide, α-silicon, etc), azo pigments, perylene pigments, anthanthrone pigments, phthalocyanine pigments, indigo pigments, triphenylmethane pigments, therene pigments, toluidine pigments, pyrazoline pigments, quinacridon pigments, dithioketopyrrolopyrrole pigments and the like. These charge generating materials can be used alone or in combination thereof according to the range of sensitivity of the electrophotosensitive material.

The benzidine derivatives represented by the general formulas (3), (3') and (5) as the hole transferring material can be used alone or in combination with the other charge transferring materials which have hitherto been known.

Examples of the other charge transferring material include various electron transferring materials and hole transferring materials.

Examples of the electron transferring material include electron attractive materials such as diphenoxy compounds, benzoquinone compounds, naphthoquinone compounds, malononitrile, thiopyran compounds, tetracyanoethylene, tetracyanoquinodimethane, chloroanil, bromoanil, 2,4,7-trinitro-9-fluorenone, 2,4,5,7-tetranitro-9-fluorenone, 2,4,7-trinitro-9-dicyanomthylenefluorenone, 2,4,5,7-tetranitroxanthone, 2,4,8-trinitrothioxanthone, dinitrobenzene, dinitroanthracene, dinitroacridine, nitroanthraquinone, dinitroanthraquinone, succinic anhydride, maleic anhydride, dibromomaleic anhydride, etc., high-molecular electron attractive materials and the like.

Examples of the hole transferring material include electron donative materials such as nitrogen-containing cyclic compounds and condensed polycyclic compounds which include diamine compounds other than benzidine derivatives represented by the general formulas (3), (3') and (5); diazole compounds such as 2,5-bis(4-methylaminophenyl)-1,3,4-oxadiazole, etc.; styryl compounds such as 9-(4-diethylaminostyryl)anthracene, etc.; carbazole compounds such as polyvinyl carbazole, etc.; pyrazoline compounds such as 1-phenyl-3-(p-dimethylaminophenl)pyrazoline, etc.; hydrazone compounds; triphenylamine compounds; indol compounds; oxazole compounds; isooxazole compounds, thiazole compounds; thiadiazole compounds; imidazole compounds; pyrazole compounds; triazole compounds and the like.

Further, when using a charge transferring material having film forming properties such as polyvinyl carbazole, the binding resin is not necessarily required.

Examples of the binding resin include thermoplastic resins such as styrene polymer, styrene-butadiene copolymer, styrene-acrilonitrile copolymer, styrene-maleic acid copolymer, acrylic polymer, styrene-acrylic copolymer, polyethylene, ethylene-vinyl acetate copolymer, chlorinated polyethylene, polyvinyl chloride, polypropylene, vinyl chloride-vinyl acetate copolymer, polyester, alkyd resin, polyamide, polyurethane, polycarbonate, polyarylate, polysulfon, diaryl phthalate resin, ketone resin, polyvinyl butyral resin, polyether resin, etc.; crosslinking thermosetting resins such as silicone resin, epoxy resin, phenol resin, urea resin, melamine resin, etc.; photosetting resins such as epoxy-acrylate, urethane-acrylate, etc. These binding resins can be used alone or in combination thereof.

Additives such as sensitizers, fluorene compounds, antioxidants, ultraviolet absorbers, plasticizers, surfactants, leveling agents, etc. can be added to the photosensitive layer, in addition to the above respective components. In order to improve sensitivity of the electrophotosensitive material, there may be used sensitizers such as tert-phenyl, halonaphthoquinones, acenaphthylene, etc. in combination with the charge generating material.

In the multi-layer electrophotosensitive material, the charge generating material constituting the charge generating layer and the binding resin can be used in various proportions. It is preferred that 5 to 1000 parts by weight, particularly 30 to 500 parts by weight of the charge generating material is used, based on 100 parts by weight of the binding resin.

The charge transferring material constituting the charge transferring layer and the binding resin can be used in various proportions within such a range as not to prevent the transmission of the charge and as to prevent the crystallization of the charge transferring material. It is preferred that 10 to 500 parts by weight, particularly 25 to 200 parts by weight of the charge transferring material containing the benzidine derivatives represented by the general formulas (3), (3') and (5) as the hole transferring material is used, based on 100 parts by weight of the binding resin so as to easily transfer the charge generated in the charge generating layer due to light irradiation.

Regarding the thickness of the multi-layer type photosensitive layer, it is preferred that the thickness of the charge generating layer is about 0.01 to 5 µm, particularly about 0.1 to 3 µm and the thickness of the charge transferring layer is about 2 to 100 µm, particularly about 5 to 50 µm.

In the single-layer type electrophotosensitive material, it is suitable that 0.1 to 50 parts by weight, particularly 0.5 to 30 parts by weight of the charge generating material and 10 to 500 parts by weight, particularly 25 to 200 parts by weight of the hole transferring material containing the benzidine derivatives represented by the general formulas (3), (3') and (5) are used, based on 100 parts by weight of the binding resin. When using in combination with the electron transferring material, it is preferred to use the electron transferring material in the amount of 5 to 100 parts by weight, particularly 10 to 80 parts by weight, based on 100 parts by weight of the binding resin. It is preferred that the total amount of the hole transferring and electron transferring materials is 20 to 500 parts by weight, particularly 30 to 200 parts, based on 100 parts by weight of the binding resin.

A barrier layer (undercoat layer) may be formed, in such a range as not to injure the characteristics of the electrophotosensitive material, between the conductive substrate and the photosensitive layer in the single-layer type electrophotosensitive material, or between the conductive substrate and the charge generating layer, between the conductive substrate and the charge transferring layer or between the charge generating layer and the charge transferring layer in the multi-layer type electrophotosensitive material. Further, a protective layer may be formed on the surface of the electrophotosensitive material.

As the conductive substrate on which the above respective layers are formed, various materials having conductivity can be used, and examples thereof include metals such as aluminum, copper, tin, platinum, silver, iron, vanadium, molybdenum, chromium, cadmium, titanium, nickel, palladium, indium, stainless steel, brass, etc.; plastic materials vapor-deposited or laminated with the above metal; glass materials coated with aluminum iodide, tin oxide, indium oxide, etc.

The conductive substrate may be made in the form of a sheet or a drum. The substrate itself may have conductivity or only the surface of the substrate may have conductivity. It is preferred that the conductive substrate has a sufficient mechanical strength when used.

When the above respective layers constituting the electrophotosensitive material are formed by a coating method, the charge generating material, the charge transferring material, the binding resin, etc. may be dispersed/mixed with a suitable solvent by a known method, for example, using a roll mill, a ball mill, an atriter, a paint shaker, a supersonic dispenser, etc. to prepare a coating solution, which is applied by a known means and then allowed to dry.

As the solvent for preparing the coating solution, there can be used various organic solvents, and examples thereof include alcohols such as methanol, ethanol, isopropanol, butanol, etc.; aliphatic hydrocarbons such as n-hexane, octane, cyclohexane, etc.; aromatic hydrocarbons such as benzene, toluene, xylene, etc.; halogenated hydrocarbons such as dichloromethane, dichloroethane, carbon tetrachloride, chlorobenzene, etc.; ethers such as dimethyl ether, diethyl ether, tetrahydrofuran, ethylene glycol dimethyl ether, diethylene glycol dimethyl ether, etc.; ketones such as acetone, methyl ethyl ketone, cyclohexanone, etc.; esters such as ethyl acetate, methyl acetate, etc.; dimethylformaldehyde, dimethylformamide, dimethylsulfoxide, etc. These solvents may be used alone or in combination thereof.

In order to improve dispersibility of the charge transferring material and charge generating material as well as smoothness of the surface of the photosensitive layer, there may be used surfactants, leveling agents, etc.

As described above, the benzidine derivative of the present invention has excellent compatibility with the binding resin while maintaining high hole transferring capability, and has a high melting point in comparison with a conventional benzidine derivative. Therefore, when it is dispersed in the binding resin, the glass transition temperature of the obtained membrane can become higher, thereby improving durability, heat resistance, etc. of the membrane. Accordingly, the benzidine derivative of the present invention can be suitably used as a hole transferring material in applications such as solar battery, electroluminescent device, electrophotosensitive material and the like.

Further, the electrophotosensitive material of the present invention has high sensitivity and is superior in durability and heat resistance.

### EXAMPLES

The following Synthesis Examples, Examples and Comparative Examples further illustrate the present invention in detail.

### Synthesis Example 1 (Reference)

14.8 g of N,N'-diacetyl-3,3'-dimethylbenzidine, 23.2 g of 3,4-dimethyliodobenzene, 13.8 g of potassium carbonate and 1 g of copper powder were added in 150 ml of nitrobenzene, and the mixture was refluxed with vigorous stirring while a nitrogen gas was bubbling into this reaction system for 24 hours. The water produced in the reaction was removed out of the reaction system by azeotropic distillation with nitrobenzene.

After the reaction solution was cooled, the inorganic substance was filtered off. Further, nitrobenzene was distilled off by steam distillation to give the residue, which was dissolved in 100 ml of tetrahydrofuran together with 10 % hydrochloric acid. The solution was deacetylated under reflux for 2 hours to give N,N'-bis(3,4-dimethylphenyl)-3,3'-dimethylbenzidine.

Then, 10.5 g of N,N'-bis(3,4-dimethylphenyl)-3,3'-dimethylbenzidine, 13.0 g of 4-n-butyliodobenzene, 13.8 g of potassium carbonate and 1 g of copper powder were added in 150 ml of nitrobenzene, and the mixture was refluxed with vigorous stirring while a nitrogen gas was blowing into this reaction system for 24 hours. The water produced in the reaction was removed out of the reaction system by azeotropic distillation with nitrobenzene, similar to the above case.

After the reaction solution was cooled, the inorganic substance was filtered off. Further, nitrobenzene was distilled off by steam distillation to give the residue, which was dissolved in 100 ml of cyclohexane and the solution was purified by subjecting to silica gel chromatography and cyclohexane was distilled off to give a white precipitation. Then, the white precipitation was recrystallized from n-hexane to give N,N'-bis(3,4-dimethylphenyl)-N,N'-bis(n-butylphenyl)-3,3'-dimethylbenzidine of the above formula (1-a) as an objective product (8.7 g, yield: 27.3 %).

The results of the infrared spectroscopic analysis of the resulting product are shown in Fig. 1.

| Elemental analysis (%), | | | |
|---|---|---|---|
| Calcd. | C, 87.65; | H, 8.26; | N, 4.09 |
| Found | C, 87.60; | H, 8.24; | N, 4.14 |
| Melting point | 180.6 °C | | |

### Example 1 (Reference)

### (Single-layer type electrophotosensitive material for digital light source)

5 Parts by weight of X-type metal-free phthalocyanine as the charge generating material, 50 parts by weight of a benzidine derivative as the hole transferring material (HTM), 30 parts by weight of 3,5-dimethyl-3',5'-di-tert-butyldiphenoquinone of the formula (12): 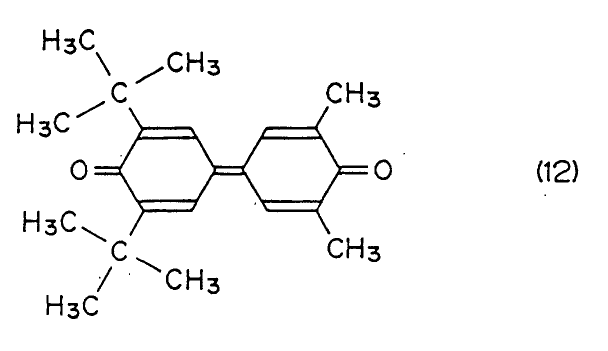 as the electron transferring material and 100 parts by weight of polycarbonate as the binding resin were mixed/dispersed with 800 parts by weight of tetrahydrofuran using a ball mill for 50 hours to prepare a coating solution for single-layer type photosensitive layer. Then, this coating solution was applied on an aluminum tube as the conductive substrate by a dip coating method, followed by hot-air drying at 100 °C for 60 minutes to give a single-layer type electrophotosensitive material for digital light source which has a single-layer type photosensitive layer of 15 to 20 µm in film thickness.

The following tests were conducted as to the electrophotosensitive material of the above Example 1 and its characteristics were evaluated.

### Initial electrical characteristics test (I)

By using a drum sensitivity tester manufactured by GENTEC Co., a voltage was applied on the surface of the electrophotosensitive material to charge the surface at +700 V. Then, monochromatic light having a wavelength of 780 nm (half-width: 20 nm) and a light intensity of 16 µW/cm ² from white light of a halogen lamp as an exposure light source through a band-pass filter was irradiated on the surface of the electrophotosensitive material for 80 msec. (irradiation time) and the time which is necessary for the above surface potential to be reduced to half, i.e. +350 V was measured, thereby calculating a half-life exposure E_{1/2} (µJ/cm²). Further, a surface potential at the time at which 330 msec. has passed since the beginning of exposure was measured as a potential after exposure V_{L} (V).

### Measurement of glass transition temperature

A photosensitive layer (about 5 mg) was peeled off from the electrophotosensitive material and the photosensitive layer was placed in an exclusive aluminum pan, which was sealed to prepare a sample to be measured. Then, the measurement was conducted as to the sample under the following conditions using a differential scanning calorimeter (type DSC8230D, manufactured by Rigaku Denki Co., Ltd.). An extrapolation glass transition temperature (Tig) of the sample was determined from the measurement results according to a "method for measuring a transition temperature of a plastic" defined in JIS K 7121.
Atmospheric gas: air
Heating rate: 20 °C/minute

### High-temperature resistance test

The electrophotosensitive material was attached to an imaging unit of a facsimile for normal paper (type LDC-650, manufactured by Mita Industrial Co., Ltd.) and stored at an atmospheric temperature of 50 °C for 10 days at a state where a cleaning blade was always pressure-contacted on the surface of the electrophotosensitive material at a linear pressure of 1.5 g/mm. Then, a state of the surface of the photosensitive layer was measured with an utility surface profile-measuring apparatus (type SE-3H, manufactured by Kosaka Kenkyusho Co., Ltd.) and a maximum depth of the resulting dent was recorded. In the following tables, the description "<0.3 µm" in the column of the dent means that no dent was observed at all because the surface roughness of a normal electrophotosensitive material having no dent is about 0.5 µ.

### Example 2 (Reference)

### (Single-layer type electrophotosensitive material for analogue light source)

5 Parts by weight of any one of azo pigments represented by the formulas: 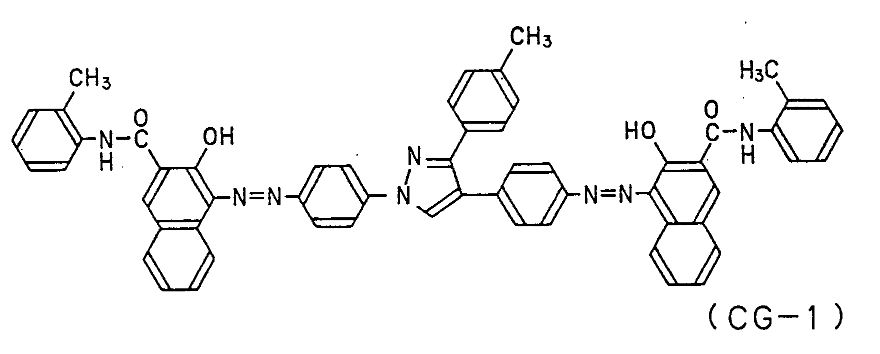 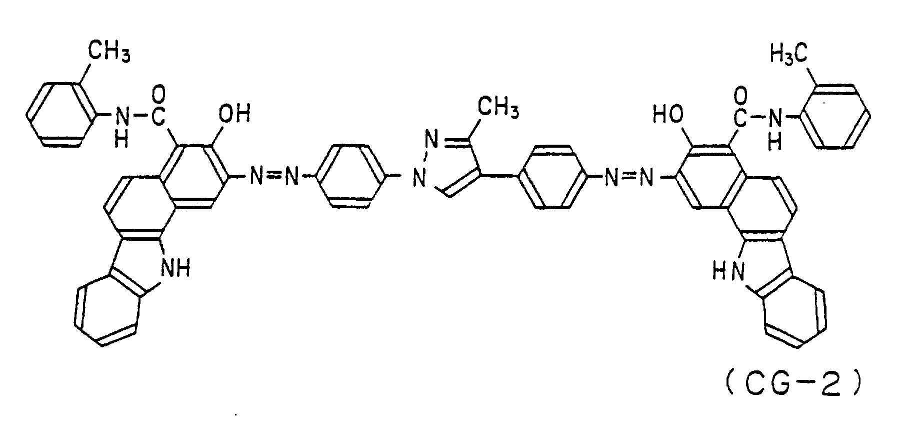 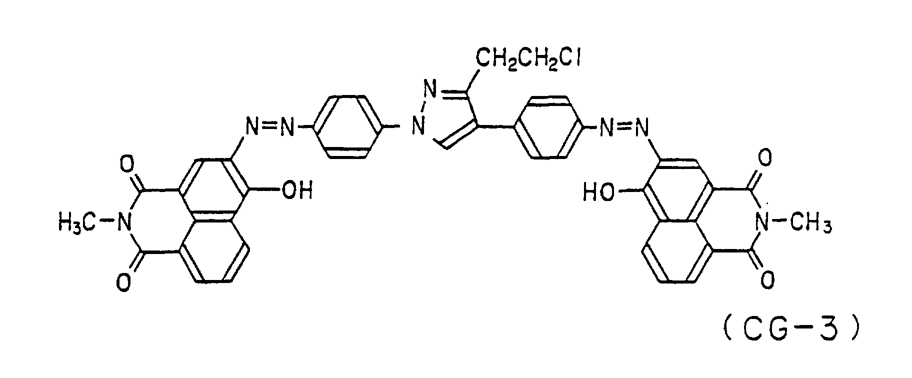 as the charge generating material (CGM), 70 parts by weight of a benzidine derivative as the hole transferring material (HTM), 20 parts by weight of 3,5-dimethyl-3',5'-di-tert-butyldiphenoquinone of the above formula (12) as the electron transferring material and 100 parts by weight of polycarbonate as the binding resin were mixed/dispersed with 800 parts by weight of tetrahydrofuran using a ball mill for 50 hours to prepare a coating solution for single-layer type photosensitive layer. Then, this coating solution was applied on an aluminum tube as the conductive substrate by a dip coating method, followed by hot-air drying at 100 °C for 60 minutes to give a single-layer type electrophotosensitive material for analogue light source which has a single-layer type photosensitive layer of 15 to 20 µm in film thickness.

The following initial electrical characteristics test (II) was conducted as to the single-layer type electrophotosensitive material thus obtained, and its characteristics were evaluated.

### Initial electrical characteristics test (II)

By using a drum sensitivity tester manufactured by GENTEC Co., a voltage was applied on the surface of the electrophotosensitive material to charge the surface at +700 V. Then, white light (light intensity: 147 µW/cm²) of a halogen lamp as an exposure light was irradiated on the surface of the electrophotosensitive material for 50 msec. (irradiation time) and the time which is necessary for the above surface potential to be reduced to half, i.e. +350 V was measured, thereby calculating a half-life exposure E_{1/2} (µJ/cm²). Further, a surface potential at the time at which 330 msec. has passed since the beginning of exposure was measured as a potential after exposure V_{L} (V).

### Example 3 (Reference) (Multi-layer type electrophotosensitive material for digital light source)

2 Parts by weight of X-type metal-free phthalocyanine as the charge generating material and 1 part of polyvinyl butyral as the binding resin were mixed/dispersed with 120 parts by weight of dichloromethane using a ball mill for 50 hours to prepare a coating solution for charge generating layer. Then, this coating solution was applied on an aluminum tube by a dip coating method, followed by hot-air drying at 100 °C for 60 minutes to form a charge generating layer of 0.5 µm in film thickness.

Then, 80 parts by weight of a benzidine derivative as the hole transferring material and 100 part of polycarbonate as the binding resin were mixed/dispersed with 800 parts by weight of benzene using a ball mill for 50 hours to prepare a coating solution for charge transferring layer. Then, this coating solution was applied on the above charge generating layer by a dip coating method, followed by hot-air drying at 90 °C for 60 minutes to form a charge transferring layer of 15 µm in film thickness, thereby producing a multi-layer type electrophotosensitive material for digital light source.

The following initial electrical characteristics test (III) was conducted as to the multi-layer type electrophotosensitive material thus obtained, and its characteristics were evaluated.

### Initial electrical characteristics test (III)

By using a drum sensitivity tester manufactured by GENTEC Co., a voltage was applied on the surface of the electrophotosensitive material to charge the surface at -700 V. Then, monochromatic light having a wavelength of 780 nm (half-width: 20 nm) and a light intensity of 16 µW/cm² from white light of a halogen lamp as an exposure light source through a band-pass filter was irradiated on the surface of the electrophotosensitive material for 50 msec. (irradiation time) and the time which is necessary for the above surface potential to be reduced to half, i.e. -350 V was measured, thereby calculating a half-life exposure E_{1/2} (µJ/cm²). Further, a surface potential at the time at which 330 msec. has passed since the beginning of exposure was measured as a potential after exposure V_{L} (V).

### Synthesis Example 2 (Reference)

16.1 g of N,N'-diacetyl-3,3',5,5'-tetramethylbenzidine, 21.8 g of p-iodotoluene, 13.8 g of potassium carbonate and 1 g of copper powder were added to 150 ml of nitrobenzene, and the mixture was refluxed with vigorous stirring while nitrogen gas was bubbling into this reaction system for 24 hours. The water produced in the reaction was removed out of the reaction system by azeotropic distillation with nitrobenzene.

After the reaction solution was cooled, the inorganic substance was filtered off. Further, nitrobenzene was distilled off by steam distillation to give the residue, which was dissolved in 100 ml of tetrahydrofuran together with 10 % hydrochloric acid. The solution was deacetylated under reflux for 2 hours to give N,N'-di(4-methylphenyl)-3,3',5,5'-tetramethylbenzidine.

Then, 10.3 g of N,N'-di(4-methylphenyl)-3,3',5,5'-tetramethylbenzidine, 13.0 g of 4-tert-butyliodobenzene, 13.8 g of potassium carbonate and 1 g of copper powder were added in 150 ml of nitrobenzene, and the mixture was refluxed with vigorous stirring while a nitrogen gas was blowing into this reaction system for 24 hours. The water content produced in the reaction was removed out of the reaction system by azeotropic distillation with nitrobenzene, similar to the above case.

After the reaction solution was cooled, the inorganic substance was filtered off. Further, nitrobenzene was distilled off by steam distillation to give the residue, which was dissolved in cyclohexane and the solution was purified by subjecting to silica gel chromatography and cyclohexane was distilled off to give a white precipitation. Then, the white precipitation was recrystallized from n-hexane to give N,N'-di(4-methylphenyl)-N,N'-di(4-tert-butylphenyl)-3,3',5,5'-tetramethylbenzidine of the formula (3-b) below as an objective product (6.71 g, yield: 39.2 %).

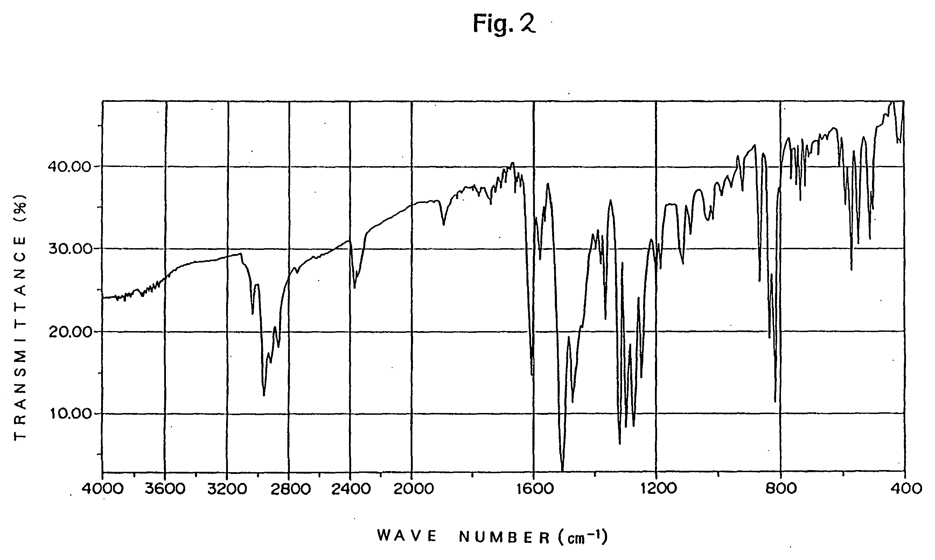

The results of the infrared spectroscopic analysis of the resulting product are shown in Fig. 2.

| Elemental analysis (%), | | | |
|---|---|---|---|
| Calcd. | C, 87.67; | H, 8.24; | N, 4.09 |
| Found | C, 87.60; | H, 8.20; | N, 4.20 |
| Melting point | 276.0 °C | | |

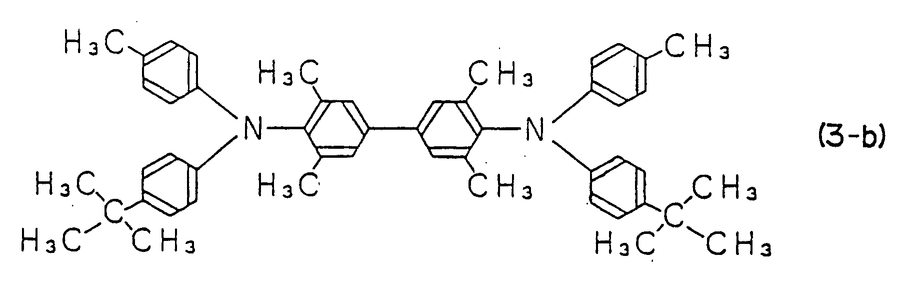

### Synthesis Example 3

According to the same manner as that described in Synthesis Example 2 except for using the same molar amount of 4-methyliodobenzene in place of p-iodotoluene and using the same molar amount of 4-ethyl-4'-iodobiphenyl in place of 4-tert-butyliodobenzene, N,N'-bis(4-methylphenyl)-N,N'-bis(4'-ethylbiphenyl-4-yl)-3,5,3',5'-tetramethylbenzidine of the above formula (3-e) was obtained (9.2 g, yield:25.0 %).

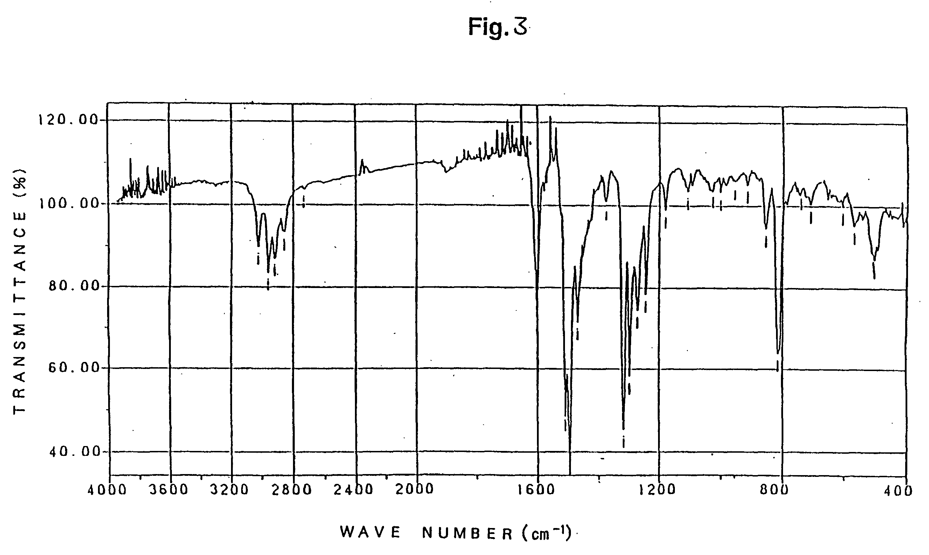

The results of the infrared spectroscopic analysis of the resulting product are shown in Fig. 3.

| Elemental analysis (%), | | | |
|---|---|---|---|
| Calcd. | C, 89.17; | H, 7.24; | N, 3.59 |
| Found | C, 89.10; | H, 7.19; | N, 3.61 |
| Melting point | 181.6 °C | | |

### Synthesis Examples 4 and 5

According to the same manner as that described in Synthesis Example 2, benzidine derivatives of the formulas (3-d) and (3-f) were obtained, respectively, using a suitable starting material.

| (Number of compound) | (Melting point) |
|---|---|
| (3-d) | 198.7 °C |
| (3-f) | 200.7 °C |

### Examples 4 to 6 and Comparative Examples 1 to 4

### (Single-layer type electrophotosensitive material for digital light source)

According to the same manner as that described in Example 1 except for using a benzidine derivative described in Table 1 as the hole transferring material (HTM), an electrophotosensitive material having a single-layer type photosensitive layer of 15 to 20 µm in film thickness.

The benzidine derivatives used in the Examples were shown by the number of the compound in Table 1. Further, benzidine derivatives (13), (18), (19), (26) and (27) used in Comparative Examples 1 to 20 are the following compounds.

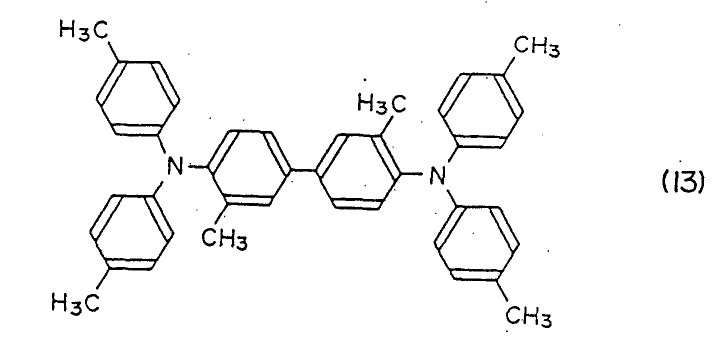

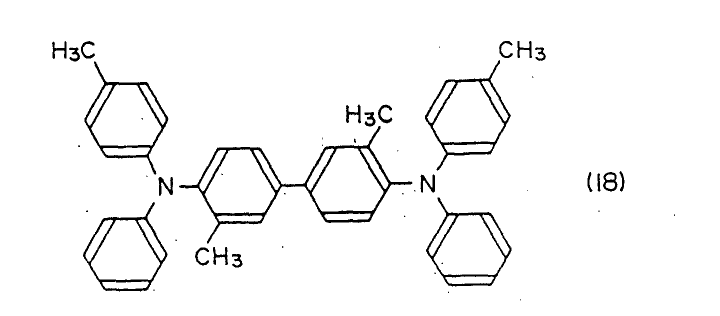

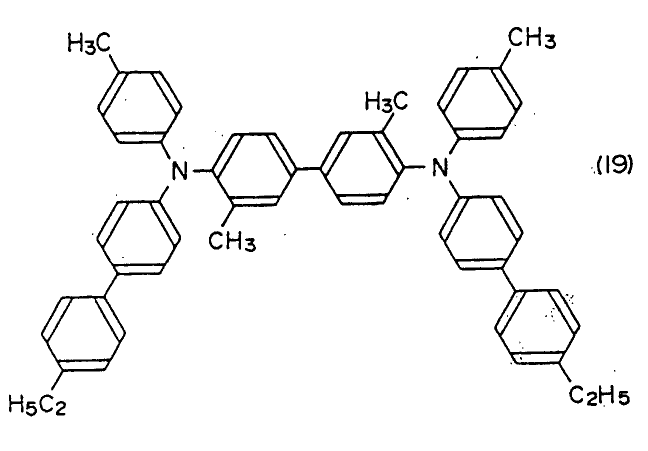

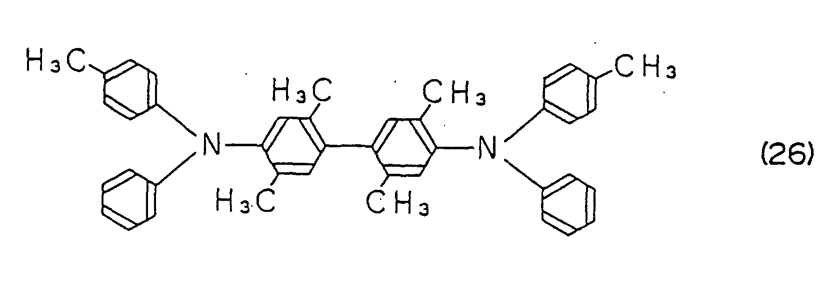

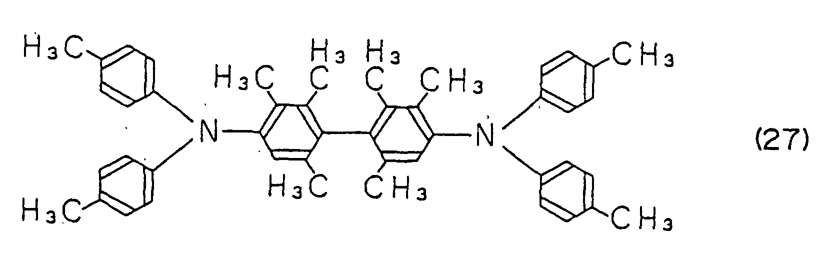

The melting point of benzidine derivatives (13), (18), (19), (26) and (27) are shown below, respectively.

| (Number of compound) | (Melting point) |
|---|---|
| (13) | 171.0°C |
| (18) | 170.1°C |
| (19) | 135.6°C |
| (26) | 224.1 °C |
| (27) | 301.5 °C |

According to the same manner as that described in Example 1, the resulting electrophotosensitive materials of the respective Examples and Comparative Examples were subjected to the initial electrical characteristics test (I), the measurement of glass transition temperature and the high-temperature resistance test. The results are shown in Table 1. Further, the test results of electrophotosensitive materials using benzidine derivatives (13) and (18) are also shown, for comparison.

**Table 1**

| | HTM | V_{L} (V) | E_{1/2} (µJ/cm²) | Tig (°C) | Dent (µm) |
|---|---|---|---|---|---|
| Example 4 | 3-d | 181 | 0.73 | 77.4 | < 0.3 |
| Example 5 | 3-e | 180 | 0.72 | 77.3 | < 0.3 |
| Example 6 | 3-f | 183 | 0.73 | 77.6 | < 0.3 |
| Comp. Example 1 | (26) | 226 | 0.99 | 74.1 | < 0.3 |
| Comp. Example 2 | (27) | _* | _ | _ | _ |
| Comp. Example 3 | (13) | 218 | 0.90 | 65.3 | 1.8 |
| Comp. Example 4 | (18) | 245 | 1.17 | 65.6 | 1.6 |

| | | | | | |
|---|---|---|---|---|---|
| *It was imposible to conduct measurement due to crystallization. | | | | | |

### Examples 7 to 15 and Comparative Examples 5 to 16

### (Electrophotosensitive material for analogue light source)

According to the same manner as that described in Example 2 except for using a benzidine derivative described in Tables 2 and 3 as the hole transferring material (HTM), a single-layer type electrophotosensitive material for analogue light source was produced. The resulting electrophotosensitive materials of the respective Examples and Comparative Examples were subjected to the same initial electrical characteristics test (II) as that of Example 2, and their characteristics were evaluated. The results are shown in Tables 2 and 3. Further, the test results of electrophotosensitive materials using benzidine derivatives (13) and (18) are also shown, for comparison.

**Table 2**

| | HTM | CGM | V_{L} (V) | E_{1/2} (µJ/cm²) |
|---|---|---|---|---|
| Comp. Example 5 | (26) | CG-1 | 217 | 4.51 |
| Comp. Example 6 | (27) | CG-1 | _* | _ |
| Comp. Example 7 | (13) | CG-1 | 202 | 4.25 |
| Comp. Example 8 | (26) | CG-2 | 218 | 4.50 |
| Comp. Example 9 | (27) | CG-2 | _* | _ |
| Comp. Example 10 | (13) | CG-2 | 210 | 4.32 |
| Comp. Example 11 | (26) | CG-3 | 216 | 4.52 |
| Comp. Example 12 | (27) | C G-3 | _* | _ |
| Comp. Example 13 | (13) | CG-3 | 220 | 4.41 |

| | | | | |
|---|---|---|---|---|
| * It was imposible to conduct measurement due to crystallization. | | | | |

**Table 3**

| | HTM | CGM | V_{L} (V) | E_{1/2} (µJ/cm²) |
|---|---|---|---|---|
| Example 7 | 3-d | CG-1 | 179 | 3.62 |
| Example 8 | 3-e | CG-1 | 177 | 3.64 |
| Example 9 | 3-f | CG-1 | 180 | 3.65 |
| Comp. Example 7 | (13) | CG-1 | 202 | 4.25 |
| Comp. Example 14 | (18) | CG-1 | 237 | 5.63 |
| Example 10 | 3-d | CG-2 | 172 | 3.59 |
| Example 11 | 3-e | CG-2 | 175 | 3.61 |
| Example 12 | 3-f | CG-2 | 174 | 3.63 |
| Comp. Example 10 | (13) | CG-2 | 210 | 4.32 |
| Comp. Example 15 | (18) | CG-2 | 240 | 5.63 |
| Example 13 | 3-d | CG-3 | 180 | 3.61 |
| Example 14 | 3-e | CG-3 | 179 | 3.64 |
| Example 15 | 3-f | CG-3 | 182 | 3.67 |
| Comp. Example 13 | (13) | CG-3 | 220 | 4.41 |
| Comp. Example 16 | (18) | CG-3 | 239 | 5.64 |

### Examples 16 and 17

### (Multi-layer type electrophotosensitive material for digital light source)

According to the same manner as that described in Example 3 except for using those shown in Table 4 as the hole transferring material (HTM), a multi-layer type electrophotosensitive material for digital light source was produced.

According to the same manner as that described in Example 2, the resulting electrophotosensitive materials were subjected to the initial electrical characteristics test (II), and their characteristics were evaluated. The test results are shown in Table 4.

**Table 4**

| | HTM | V_{L} (V) | E_{1/2} (µJ/cm²) |
|---|---|---|---|
| Example 16 | 3-d | -138 | 0.61 |
| Example 17 | 3-e | -135 | 0.62 |

### Synthesis Example 6

10.6 g of 3,3'-dimethylbenzidine, 33.9 g of 4-ethyl-4'-iodobiphenyl, 27.6 g of potassium carbonate and 2 g of copper powder were added to 150 ml of nitrobenzene, and the mixture was refluxed with vigorous stirring while a nitrogen gas was bubbling into this reaction system for 24 hours. The water produced in the reaction was removed out of the reaction system by azeotropic distillation with nitrobenzene.

After the reaction solution was cooled, the inorganic substance was filtered off. Further, nitrobenzene was distilled off by steam distillation to give the residue, which was dissolved in cyclohexane and the solution was purified by subjecting to silica gel chromatography and cyclohexane was distilled off to give a white precipitation. Then, the white precipitation was recrystallized from n-hexane to give N,N, N',N'-tetrakis(4'-ethylbiphenyl-4-yl)-3,3'-dimethylbenzidine of the above formula (5-b) (14.7 g, yield: 31.5 %).

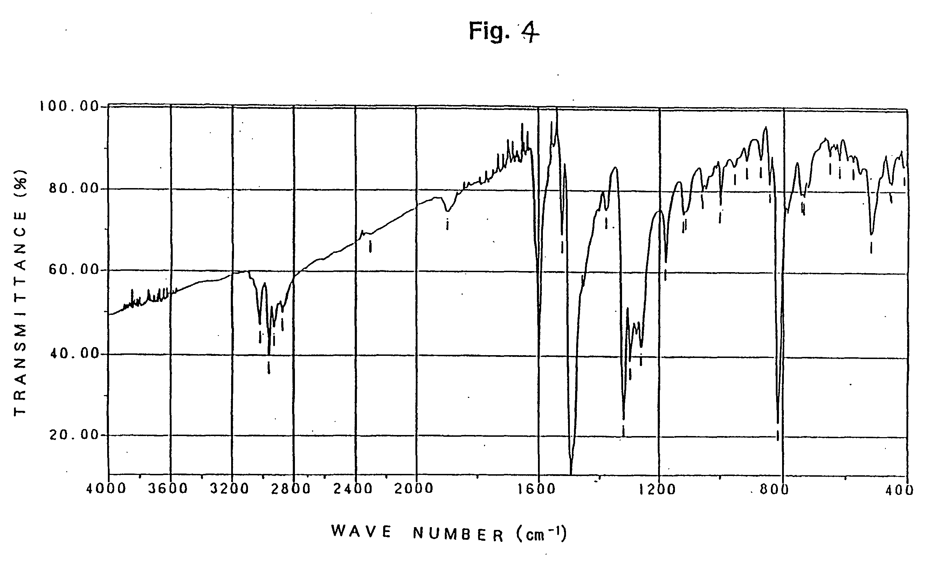

The results of the infrared spectroscopic analysis of the resulting product are shown in Fig. 4

| Elemental analysis (%), | | | |
|---|---|---|---|
| Calcd. | C, 90.07; | H, 6.93; | N, 3.00 |
| Found | C, 89.98; | H, 6.91; | N, 3.01 |
| Melting point | 270.4 °C | | |

### Synthesis Examples 7 and 8

According to the same manner as that described in Synthesis Example 6, benzidine derivatives of the formulas (5-a) and (5-c) were obtained using a suitable starting material.

| (Number of compound) | (Melting point) |
|---|---|
| (5-a) | 203.3 °C |
| (5-c) | 281.3 °C |

### Examples 18 to 20

### (Single-layer type electrophotosensitive material for digital light source)

According to the same manner as that described in Example 1 except for using a benzidine derivative shown by the number of the compound in Table 5 as the hole transferring material (HTM), a single-layer type electrophotosensitive material for digital light source was produced. The resulting electrophotosensitive materials of the above Examples and Comparative Examples were subjected to the initial electrical characteristics test (II), the measurement of the glass transition temperature and the high-temperature resistance test, and their characteristics were evaluated. The results are shown in Table 5. Further, the test results of electrophotosensitive materials using benzidine derivatives of the formulas (13), (18) and (19) are also shown, for comparison.

**Table 5**

| | HTM | V_{L} (V) | E_{1/2} (µJ/cm²) | Tig (°C) | Dent (µm) |
|---|---|---|---|---|---|
| Example 18 | 5-a | 171 | 0.70 | 76.1 | < 0.3 |
| Example 19 | 5-b | 164 | 0.65 | 75.2 | < 0.3 |
| Example 20 | 5-c | 174 | 0.69 | 76.2 | < 0.3 |
| Comp. Example 3 | (13) | 218 | 0.90 | 65.3 | 1.8 |
| Comp. Example 4 | (18) | 245 | 1.17 | 65.6 | 1.6 |
| Comp. Example 17 | (19) | 178 | 0.70 | 68.4 | 1.1 |

### Examples 21 to 29

### (Electrophotosensitive material for analogue light source)

According to the same manner as that described in Example 2 except for using a benzidine derivative described in Table 6 as the hole transferring material (HTM), a single-layer type electrophotosensitive material for analogue light source was produced. The resulting electrophotosensitive materials of the respective Examples and Comparative Examples were subjected to the same initial electrical characteristics test (II) as that of Example 2, and their characteristics were evaluated. The test results are shown in Table 6. Further, the test results of electrophotosensitive materials using benzidine derivatives of the formulas (13), (18) and (19) are also shown, for comparison.

**Table 6**

| | HTM | CGM | V_{L} (V) | E_{1/2} (µJ/cm²) |
|---|---|---|---|---|
| Example 21 | 5-a | CG-1 | 151 | 3.21 |
| Example 22 | 5-b | CG-1 | 146 | 3.17 |
| Example 23 | 5-c | CG-1 | 154 | 3.29 |
| Comp. Example 7 | (13) | CG-1 | 202 | 4.25 |
| Comp. EXAMPLE 14 | (18) | CG-1 | 237 | 5.63 |
| Comp. Example 18 | (19) | CG-1 | 152 | 3.24 |
| Example 24 | 5-a | CG-2 | 150 | 3.20 |
| Example 25 | 5-b | CG-2 | 149 | 3.19 |
| Example 26 | 5-c | CG-2 | 152 | 3.29 |
| Comp. Example 10 | (13) | CG-2 | 210 | 4.32 |
| Comp. Example 15 | (18) | CG-2 | 240 | 5.63 |
| Comp. Example 19 | (19) | CG-2 | 154 | 3.21 |
| Example 27 | 5-a | CG-3 | 148 | 3.21 |
| Example 28 | 5-b | CG-3 | 151 | 3.31 |
| Example 29 | 5-c | CG-3 | 153 | 3.20 |
| Comp. Example 13 | (13) | CG-3 | 220 | 4.41 |
| Comp. Example 16 | (18) | CG-3 | 239 | 5.64 |
| Comp. Example 20 | (19) | CG-3 | 156 | 3.23 |

### Examples 30 and 31

### (Multi-layer type electrophotosensitive material for digital light source)

According to the same manner as that described in Example 3 except for using those shown in Table 7 as the hole transferring material (HTM), a multi-layer type electrophotosensitive material for digital light source was produced.

The resulting multi-layer type electrophotosensitive materials were subjected to the initial electrical characteristics test (III) according to the same manner as that described in Example 3, and their characteristics were evaluated. The test results are shown in Table 7.

**Table 7**

| | HTM | V_{L} (V) | E_{1/2} (µJ/cm²) |
|---|---|---|---|
| Example 30 | 5-a | -120 | 0.51 |
| Example 31 | 5-b | -124 | 0.53 |

### Synthesis Example 9

According to the same manner as that described in Synthesis Example 1, 14.9 g of N,N'-diacetyl-3,3'-dimethylbenzidine was reacted with 21.8 g of para-iodobenzene to give N,N'-di-p-tolyl-3,3'-dimethylbenzidine. Further, 12.0 g of N,N'-di-p-tolyl-3,3'-dimethylbenzidine was reacted with 15.4 g of 4-ethyl-4'-iodobiphenyl according to the same manner as that described in Synthesis Example 1 to give N,N'-di-p-tolyl-N,N'-di(4'-ethylbiphenyl-4-yl)-3,3'-dimethylbenzidine (hereinafter referred to as 6-c) (8.82 g, yield: 23.7 %).

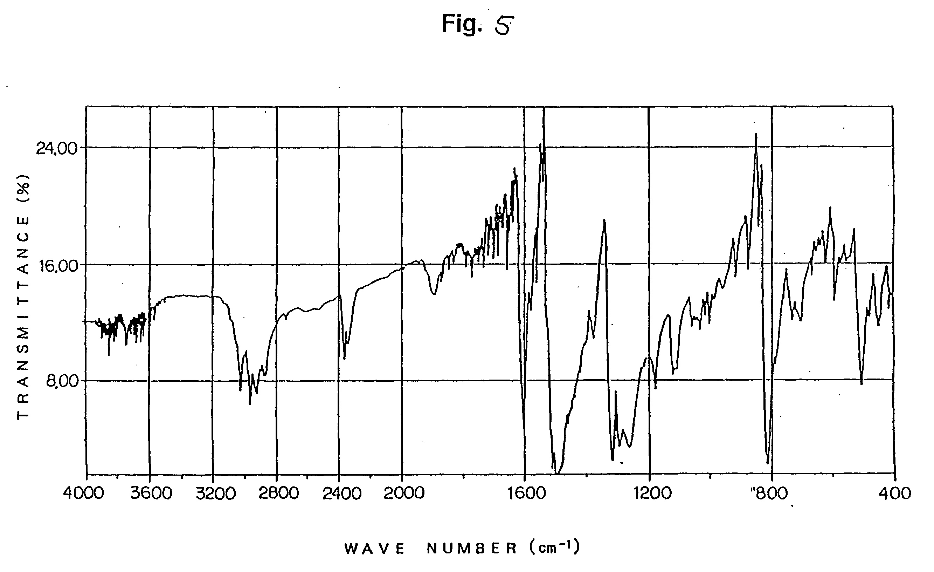

The results of the infrared spectroscopic analysis of the above compound 6-c are shown in Fig. 5.

| Elemental analysis (%), | | | |
|---|---|---|---|
| Calcd. | C, 89.32; | H, 6.96; | N, 3.72 |
| Found | C, 88.99; | H, 6.90; | N, 3.90 |
| Melting point | 135.6 °C | | |

### Synthesis Example 10

According to the same manner as that described in Synthesis Example 9 except for using 4-iodobiphenyl in place of 4-ethyl-4'-iodobiphenyl, N,N'-di-p-tolyl-N,N'-di(biphenyl-4-yl)-3,3'-dimethylbenzidine (hereinafter referred to as 6-a) was obtained.
Melting point: 181.5 °C

### Synthesis Example 11

According to the same manner as that described in Synthesis Example 9 except for using 4-methyl-4'-iodobiphenyl in place of 4-ethyl-4'-iodobiphenyl, N,N'-di-p-tolyl-N,N'-di(4-methylbiphenyl-4'-yl)-3,3'-dimethylbenzidine (hereinafter referred to as 6-b) was obtained.
Melting point: 168.3 °C

## Claims

1. A benzidine derivative represented by the general formula (3): 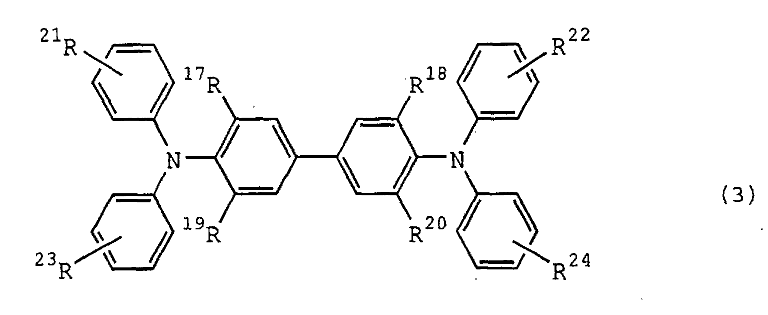 wherein, R¹⁷, R¹⁸, R¹⁹ and R ²⁰ are the same or different and indicate a C₁ to C₆ alkyl group; R²¹ and R²² are the same or different and indicate a hydrogen atom or a C₁ to C₆ alkyl group; and R²³ and R²⁴ are the same or different and indicate a phenyl, tolyl, naphthyl, biphenyl, anthryl or phenanthryl group or any thereof having a C₁ to C₆ alkyl substituent, or C₁ to C₆ alkoxy substituent or a halogen substituent.

2. The benzidine derivative according to claim 1, which is represented by the general formula (3') : 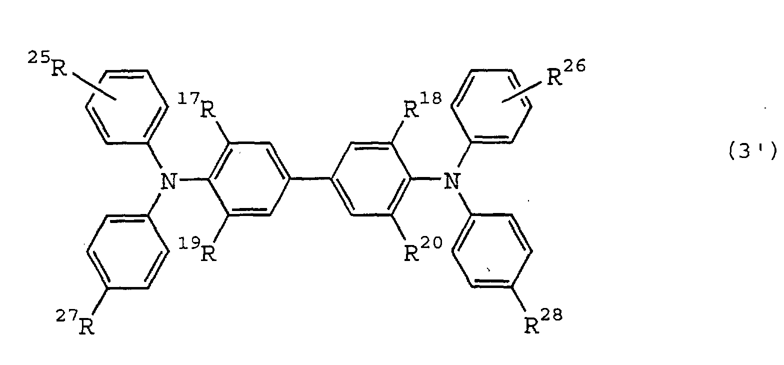 wherein R¹⁷, R¹⁸, R¹⁹ and R²⁰ are as defined in claim 1; R²⁵ and R²⁶ are the same or different and indicate a hydrogen atom or a C₁ to C₆ alkyl group; and R²⁷ and R²⁸ are the same or different and indicate a phenyl, tolyl, naphthyl, biphenyl, anthryl or phenanthryl group or any thereof having a C₁ to C₆ alkyl substituent, or C₁ to C₆ alkoxy substituent or a halogen substituent.

3. A benzidine derivative represented by the general formula (5): 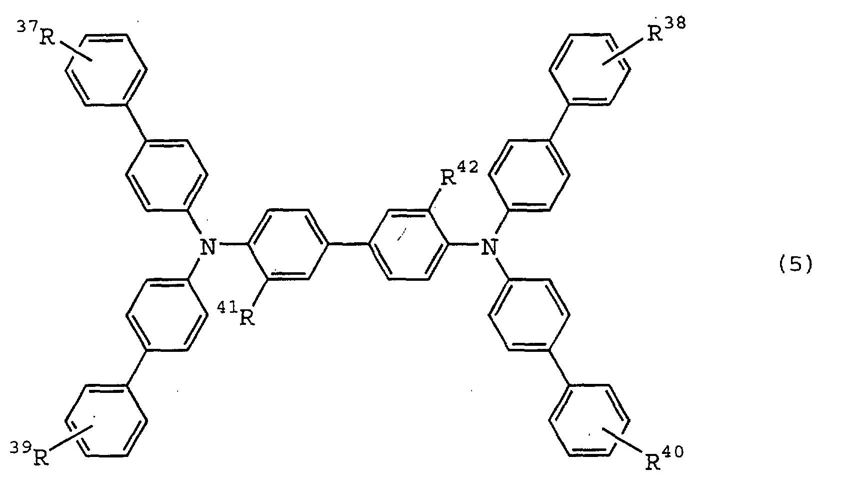 wherein R³⁷, R³⁸, R ³⁹ and R ⁴⁰ are the same or different and indicate a hydrogen atom or a C₁ to C₆ alkyl group; and R⁴¹ and R⁴² are the same or different and indicate a C₁ to C₆ alkyl group.

4. An electrophotosensitive material comprising a conductive substrate, and a photosensitive layer provided on the conductive substrate, said photosensitive layer containing the benzidine derivative represented by the general formula (3) of claim 1 , the general formula (3') of claim 2 or the general formula (5) of claim 3.

## Patentansprüche

1. Benzidin-Derivat der allgemeinen Formel (3): 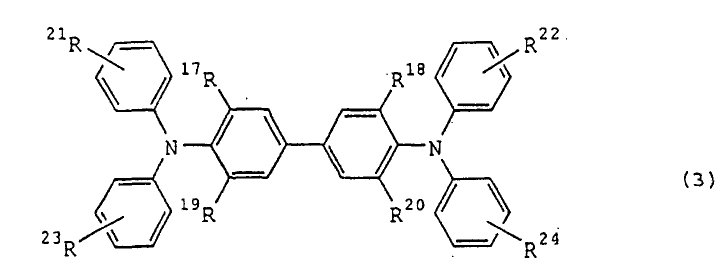 in der R¹⁷, R¹⁸, R¹⁹ und R²⁰ gleich oder verschieden sind und eine C₁-C₆-Alkyl-Gruppe bedeuten, R²¹ und R²² gleich oder verschieden sind und ein Wasserstoffatom oder eine C₁-C₆-Alkyl-Gruppe bedeuten und R²³ und R²⁴ gleich oder verschieden sind und eine Phenyl-, Tolyl-, Naphthyl-, Biphenyl-, Anthryl- oder Phenanthryl-Gruppe bedeuten oder eine beliebige dieser Gruppen mit einem C₁-C₆-Alkyl-Substituenten oder C₁-C₆-Alkoxy-Substituenten oder einem Halogen-Substituenten

2. Benzidin-Derivat nach Anspruch 1, mit der allgemeinen Fomel (3') 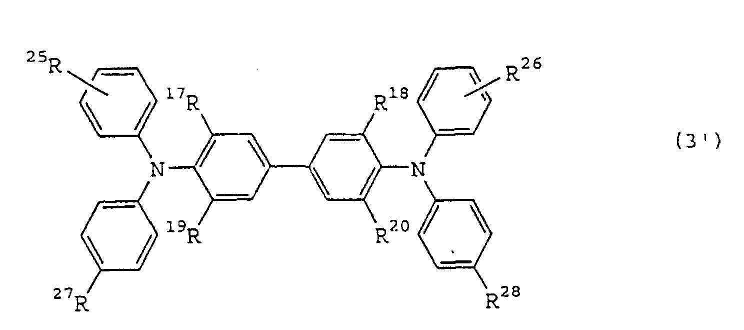 in der R¹⁷, R¹⁸, R¹⁹ und R²⁰ wie in Anspruch 1 definiert sind, R²⁵ und R²⁶ gleich oder verschieden sind und ein Wasserstoffatom oder eine C₁-C₆-Alkyl-Gruppe bedeuten und R²⁷ und R²⁸ gleich oder verschieden sind und eine Phenyl-, Tolyl-, Naphthyl-, Biphenyl-, Anthryl- oder Phenanthryl-Gruppe bedeuten oder eine beliebige dieser Gruppen mit einem C₁-C₆-Alkyl-Substituenten oder C₁-C₆-Alkoxy-Substituenten oder einem Halogenatom-Substituenten.

3. Benzidin-Derivat der allgemeinen Formel (5): 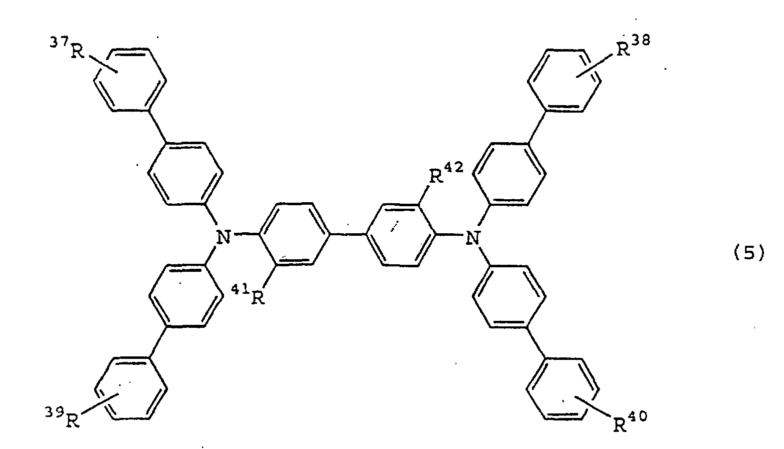 in der R³⁷, R³⁸, R³⁹ und R⁴⁰ gleich oder verschieden sind und ein Wasserstoffatom oder eine C₁-C₆-Alkyl-Gruppe bedeuten und R⁴¹ und R⁴² gleich oder verschieden sind und eine C₁-C₆-Alkyl-Gruppe bedeuten.

4. Elektrophotoempfindliches Material, das ein leitendes Substrat und eine auf dem leitenden Substrat vorgesehene photoempfindliche Schicht aufweist, wobei die photoempfindliche Schicht das durch die allgemeine Formel (3) in Anspruch 1, die allgemeine Formel (3') in Anspruch 2 oder die allgemeine Formel (5) in Anspruch 3 dargestellte Benzidin-Derivat enthält.

## Revendications

1. Un dérivé de benzidine représenté par la formule générale (3) : 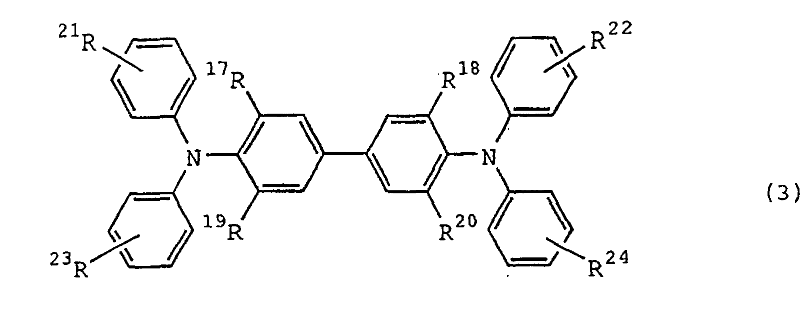 dans laquelle R¹⁷, R¹⁸, R¹⁹ et R²⁰ sont identiques ou différents et désignent un groupe alkyle en C₁-C₆ ; R²¹ et R²² sont identiques ou différents et désignent un atome d'hydrogène ou un groupe alkyle en C₁-C₆ ; et R²³ et R²⁴ sont identiques ou différents et désignent un groupe phényle, tolyle, naphtyle, diphényle, anthryle ou phénanthryle ou l'un de ceux-ci ayant un substituant alkyle en C₁-C₆, ou un substituant alcoxy en C₁-C₆ ou un substituant halogène.

2. Le dérivé de benzidine selon la revendication 1, qui est représenté par la formule générale (3') : 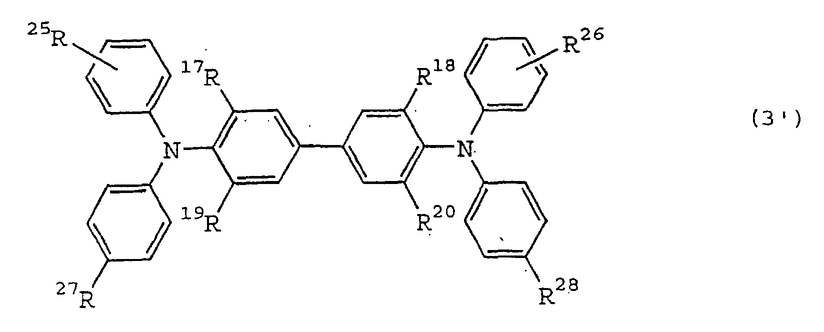 dans laquelle R¹⁷, R¹⁸, R¹⁹ et R²⁰ sont tels que définis dans la revendication 1 ; R²⁵ et R²⁶ sont identiques ou différents et désignent un atome d'hydrogène ou un groupe alkyle en C₁-C₆ ; et R²⁷ et R²⁸ sont identiques ou différents et désignent un groupe phényle, tolyle, naphtyle, diphényle, anthryle, ou phénanthryle , ou l'un quelconque de ceux-ci ayant un substituant alkyle en C₁-C₆, ou un substituant alcoxy en C₁-C₆ ou un substituant halogène.

3. Un dérivé de benzidine représenté par la formule générale (5) : 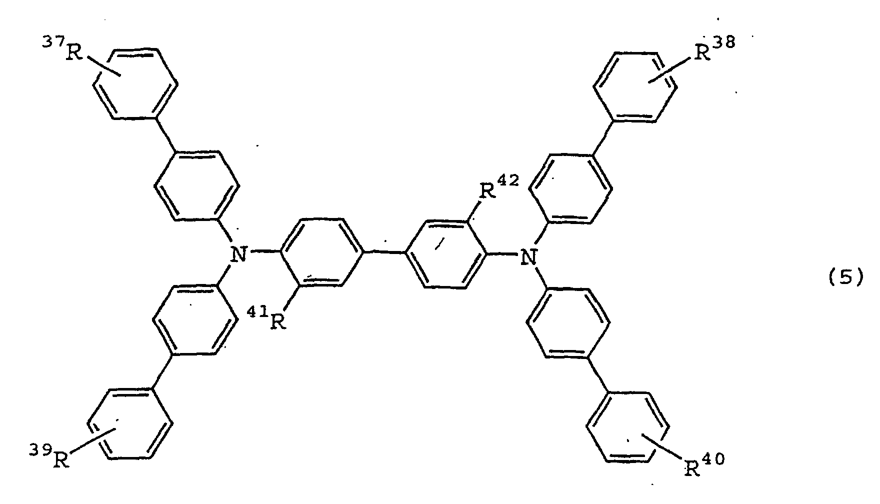 dans laquelle R³⁷, R³⁸, R³⁹ et R⁴⁰ sont identiques ou différents et désignent un atome d'hydrogène ou un groupe alkyle en C₁-C₆ ; et R⁴¹ et R⁴² sont identiques ou différents et désignent un groupe alkyle en C₁-C₆.

4. Un matériau électrophotosensible comprenant un substrat conducteur et une couche photosensible prévue sur le substrat conducteur, ladite couche photosensible contenant le dérivé de benzidine représenté la formule générale (3) de la revendication 1, la formule générale (3') de la revendication 2 ou la formule (5) de la revendication 3.
